(19) Europäisches Patentamt
European Patent Office
Office européen des brevets

(11) **EP 4 657 447 A1**

(12) **EUROPEAN PATENT APPLICATION**

(43) Date of publication:
**03.12.2025 Bulletin 2025/49**

(21) Application number: **24382580.9**

(22) Date of filing: **30.05.2024**

(51) International Patent Classification (IPC):
**G16H 10/60** (2018.01)    **G16H 50/50** (2018.01)
**G16H 50/70** (2018.01)

(52) Cooperative Patent Classification (CPC):
**G16H 50/70; G06N 3/045; G06N 3/08; G06N 20/00; G16H 10/60; G16H 50/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Roche Diabetes Care GmbH**
**68305 Mannheim (DE)**

(72) Inventors:
• **ANDORRÀ INGLÉS, Magí**
 **08174 Sant Cugat del Vallès, Barcelona (ES)**
• **HERRERO VIÑAS, Pau**
 **08174 Sant Cugat del Vallès, Barcelona (ES)**

(74) Representative: **Bittner, Thomas L.**
**Boehmert & Boehmert**
**Anwaltspartnerschaft mbB**
**Pettenkoferstrasse 22**
**80336 München (DE)**

(54) **METHOD FOR GENERATING SYNTHETIC DATA, SYNTHETIC DATA GENERATION DEVICE, COMPUTER PROGRAM PRODUCT, METHOD FOR PREDICTING A HEALTHCARE-RELEVANT PARAMETER AND DIABETES MANAGEMENT DEVICE**

(57)    A method for generating synthetic data is provided, comprising receiving, via an input device, input data comprising input first data indicative of a first healthcare-relevant parameter, the input first data providing at least one sparse time series input signal for the first healthcare-relevant parameter, and determining, in a processing device, auxiliary first data from the input first data. The sparse time series input signal of the input first data is encoded into a dense time series input signal for the first healthcare-related parameter, the auxiliary first data providing the dense time series input signal. In the processing device, synthetic data is determined from modified input data comprising the auxiliary first data such that the synthetic data closely resembles the modified input data. Further, a corresponding synthetic data generation device, a computer program product, a method for predicting a healthcare-relevant parameter, and a diabetes management device are provided.

Fig. 3

## Description

The present invention refers to a method for generating synthetic data, a synthetic data generation device, a computer program product, a method for predicting a healthcare-relevant parameter and a diabetes management device.

### Background

Synthetic data is data generated on the basis of an original dataset, the synthetic data generally having the same statistical properties (as dictated by a given application, e.g. distribution of relevant attributes and dependency between them) as the original dataset. Applications of synthetic data include providing an alternative to conventional anonymization for securing data privacy and augmenting data by generating additional data based on a limited amount of original data, for example for providing sufficient training data in machine learning applications (see Richard J Chen et al., Synthetic data in machine learning for medicine and Healthcare, Nature Biomedical Engineering, 5(6):493-497, 2021).

Different approaches for generation of synthetic data in general have been proposed, including variational autoencoders (cf. e.g. Siddharth Biswal at al., Generating longitudinal electronic health records using conditional variational autoencoders, Machine Learning for Healthcare Conference, pages 260-282, PMLR, 2021), copula-based methods (e.g. David Meyer et al., Copula-based synthetic data augmentation for machine-learning emulators, Geoscientific Model Development, 14:5205-5215, 8 2021) and generative adversarial networks (GANs, see Fabi Prezja et al., Deepfake knee osteoarthritis x-rays from generative adversarial neural networks deceive medical experts and offer augmentation potential to automatic classification, Scientific Reports, 12(1):1-16, 2022).

However, synthetic data generation is much more complex in the context of time-series, or longitudinal data. Particular challenges lie in capturing complex multidimensional relationships between heterogenous signals (e.g., mixture of continuous signals, impulse and categorical signals, cf. Fodil Benali et al., Synthetic complex data generation using copula, 23rd International Workshop on Design, Optimization, Languages and Analytical Processing of Big Data (DOLAP), pages 51-60, 2021), and capturing long-term dependencies, e.g., disease progression (see Philipp Wendland et al., Generation of realistic synthetic data using multimodal neural ordinary differential equations, NPJ digital medicine, 5(1):1-10, 2022).

In the document WO 2022/235618 A1, it is proposed to implement data augmentation using synthetic data with regard to blood glucose measurements with a given specific symptom such as hypoglycemia.

### Summary

It is an object of the present invention to provide improved techniques in generating synthetic data, in particular with regard to quality of synthetic data in the context of multivariate time-series data in healthcare applications.

To solve the problem, a method for generating synthetic data for use in healthcare applications according to the independent claim 1 is provided. Further, a synthetic data generation device, a computer program product, a method for predicting a healthcare-relevant parameter and a diabetes management device according to further independent claims are provided. Embodiments are the subject matter of dependent claims in the appended set of claims.

According to one aspect, a method for generating synthetic data for use in healthcare applications is provided. The method comprises receiving, via an input device, input data comprising input first data indicative of a first healthcare-relevant parameter, the input first data providing at least one sparse time series input signal for the first healthcare-relevant parameter. The method then comprises determining, in a processing device, auxiliary first data from the input first data, wherein the sparse time series input signal of the input first data is encoded into a dense time series input signal for the first healthcare-related parameter, the auxiliary first data providing the dense time series input signal for the first healthcare-related parameter, and determining, in the processing device, synthetic data, from modified input data comprising the auxiliary first data, such that the synthetic data closely resembles the modified input data in terms of statistical properties.

In the sense of the present disclosure, a sparse time series signal is to be understood in contrast to a dense time series signal. That is, a dense time series signal comprises a dense set of datapoints that could approximate a graph (particularly is suitable to approximate a curved shape) while a sparse time series signal comprises non-zero datapoints that is widely separated from any other non-zero datapoints in the same time series as is the case, for example, for delta impulse spikes in such data as for example indicating glucose intakes (meals) or bolus administration in diabetes therapy. In this context, it is to be understood that in relation to the present disclosure, non-zero datapoints being widely separated from any other non-zero datapoints is a definition relative to a given use case. Thus, a dense time series signal in the sense of the present disclosure may comprise a number of signal spikes that are close together in an absolute sense but are widely separated in the sense that they form spikes or discontinuities as compared to a smooth or continuous time series signal suitable.

The sparse time series signal may be understood to indicate a discrete signal while the dense time series signal may be understood to indicate a continuous signal which as a digital signal is considered quasi-continuous since true continuity cannot be achieved in the digital realm.

In mathematical term, the terms "sparse" and "dense" may be understood to refer to the number of zero vs. non-zero elements in an array (e.g. vector or matrix) of the time series signal. A sparse array is one that contains mostly zeros and few non-zero entries. A dense array contains mostly non-zeros. For example, a vector is considered k -sparse if it contains at most k non-zero entries. Another way of saying this is that the vector's $\ell 0$ norm is k. By way of example, the vector $(0,0,0,0,0,0,0,0,0,50,0,0,0,0,0,0,0,0,0,75,0,0,0,0,...,0,0,30,0,0,0)$ has l0 norm equal to 3 (i.e., 3 non zero values). Such vector is therefore denominated 3-sparse. In an exemplary embodiment, input data, in particular the input first data is between 1-sparse and 5-sparse. In one such embodiment, the input data, in particular input first data defines days of data that are between 1-sparse and 5 -sparse.

In the sense of the present disclosure, synthetic data closely resembles (modified) input data in terms of statistical properties, when the deviation in relevant statistical properties between the synthetic data and the (modified) input data is small enough to allow for successful replacement or augmentation of the (modified) input data by the synthetic data, which is the case if, for a given application, the synthetic data may be used in the same way as the (modified) input data without unacceptable differences in the results of such use.

In embodiments, close resemblance may be determined on the basis of deviation (or lack thereof) in a selection of - in a particular embodiment in all of - the following metrics: Base metrics, comprising Mean, Median, Standard deviation, Interquantile range, kruskal-wallis, f-test; Correlation metrics, comprising Mean Max Cross-Correlation, Std Max Cross-Correlation, Max Max Cross-Correlation; Glycemic metrics, comprising TIR, TBR, TAR, TAR250, tbr54, TIR140, GRI, Mean, Median, Standard deviation, Coefficient of variation, Min, Max, First quartile, Third quartile, HBGI, LBGI, RI, MAGE, MODD, J index, ADRR, CONGA24, GMI, eA1c, MAG, CVGA-A, CVGA-B, CVGA-C, CVGA-D, CVGA-E; Machine learning metrics, comprising Train real, test synthethic, Train synthetic (test real), Train augmented; Scenario Metrics, comprising CGM loss (%), CGM gap (min), Total daily insulin, Total daily carbohydrates, Number of meal per day, Bolus-basal split, Peak glucose time-B, Peak glucose level-B, Mean carbohydrates-B, Peak glucose time-L, Peak glucose level-L, Mean carbohydrates-L, Peak glucose time-D, Peak glucose level-D, Mean carbohydrates-D; Visualization Metrics, comprising PCA, t-SNE. In particular, close resemblance may be determined in case statistical similarity is determined based on a (statistical) similarity measure for the metric or combination of metrics chosen. Different similarity measures are known as such in the art and may be chosen as adequate in line with an application at hand. Non-limiting examples of such statistical similarity metrics include inverse distance metrics such as the inverse of the Euclidean, Manhattan, Minkowski, or Chebyshev distances between the centers, amplitudes, widths, and frequencies of the synthetically generated spikes compared to analogous spikes in observed data, in particular as used as input data. Other non-limiting examples of similarity metrics that take into account covariances and correlations between different variables in the relevant data may quantify statistical similarity to determine if the synthetically generated data closely resembles the relationships between two or more variables in the observed data, in particular the relevant input data.

In alternative methods for generating synthetic data covered by the present disclosure, determining, in the processing device, synthetic data, from modified input data comprising the auxiliary first data, may be performed such that the synthetic data closely resembles the modified input data in terms of other properties as an alternative or in addition to statistical properties. Such other properties may be chosen as relevant to a given use case in which the synthetic data shall replace or augment original data where the goal of replacement/augmentation dictates the properties for which close resemblance must be ensured to allow successful replacement or augmentation.

The input device may be a communication device, in particular a receiver or transceiver (e.g. radio, WiFi, Bluetooth, Infrared, or the like) configured to receive the input data. Alternatively or additionally, the input device may comprise a user interface allowing a user to input the input data.

The method may further comprise determining, in the processing device, output first data by extracting, from the synthetic data, data indicative of the first healthcare-relevant parameter providing a dense time series output signal for the first healthcare-relevant parameter and decoding the dense time series output signal for the first healthcare-relevant parameter into a sparse time series output signal, the output first data providing the sparse time series output signal for the first healthcare-relevant parameter. Thereby, the generation of synthetic data may be performed using a dense time series signal which is itself based on a sparse time series signal and a sparse time series output may be gained from the synthetic data thus obtained. Decoding the dense time series output signal for the first healthcare-relevant parameter into a sparse time series output signal may comprise generating a plurality of sparse data elements, each sparse data element being generated with a time and amplitude based on a corresponding peak time and peak amplitude in the dense time series output signal, in particular a peak time and peak amplitude of a Gaussian curve in a plurality of Gaussian curves in the synthetic data.

The synthetic data may be determined using a statistics-based method. In exemplary embodiments, the synthetic data may be determined using a copula-based method, in particular a Gaussian copula. Copulas are probabilistic models based on Sklar's theorem. The copula is a

function that combines all marginal distribution into the joint distribution. In the case of gaussian copula, the copula is based on the multivariate gaussian distribution (F) and the inverse of the Sklar's theorem and can be expressed as

$$C(u_1, \dots, u_d) = F\left(F_1^{-1}(u_1), \dots, F_1^{-1}(u_1)\right)$$

Herein, $F_i$ are the marginal distributions of the different time series. In an exemplary embodiment, the determining the synthetic data is based on a particular implementation of Gaussian copula provided by the Synthia library (see David Meyer and Thomas Nagler, Synthia: multidimensional synthetic data generation in python, Journal of Open Source Software, 6:2863, 9 2021; and David Meyer et al., Copula-based synthetic data augmentation for machine-learning emulators, Geoscientific Model Development, 14:5205-5215, 8 2021).

The synthetic data may be determined using machine learning. In exemplary embodiments, the synthetic data may be determined using generative adversarial networks (GANs). GANs that are known as such are composed by two blocks with opposite objectives: the generator and the discriminator. The discriminator is a classifier that distinguishes between real and synthetic data. In this case, the role of the generator is to map random noise to valid synthetic data. The discriminator tries to catch the generator's synthetic data and the generator tries to create synthetic data in a way that can pass the discriminator, hence they are competing. In an exemplary embodiment, the determining the synthetic data is based on a GAN architecture referred to as DoppleGANger (see Zinan Lin et al., Using GANs for sharing networked time series data: Challenges, initial promise, and open questions, Proceedings of the ACM Internet Measurement Conference, pages 464-483, 2020). While DoppleGANger proposes a framework to generate synthetic time series with matched metadata, in exemplary embodiments, only the sub-module of this architecture that generates time series may be employed in generating the synthetic data. The generator may be based on the Long Short-Term Memory (LSTM) cell and may use the Wasserstein loss with gradient penalty to optimize the weights (cf. Martin Arjovsky et al., Wasserstein GAN, arXiv:1701.07875v3 [stat.ML], 6 December 2017).

Determining the auxiliary first data may comprise encoding the sparse time series input signal of the input first data into a dense time series input signal for the first healthcare-related parameter using Kernel Convolution and/or at least one pharmacokinetic model. Kernel convolution is as such a well know operation in signal processing.

In the case of using Kernel Convolution, the chosen kernel may be a normalized gaussian kernel (which, of note and in line with the known art, is different from Gaussian copula as referred to elsewhere in the present disclosure), for example with a standard deviation of 10 and truncated for values beyond 10 standard deviations. With the kernel, the convolution with the delta signal may be a gaussian shifted to the time when an instance of the sparse time series signal (e.g. an impulse) occurs and with the amplitude of the instance of the sparse time series signal. For example, the at least one sparse time series input signal for the first healthcare-relevant parameter may indicate as an instance a meal ingestion and the amplitude may be an amount of carbohydrates ingested in grams. In alternative embodiments, other kernels than a gaussian kernel may be used, for example Butterworth or staircase.

Decoding the dense time series output signal for the first healthcare-relevant parameter into a sparse time series output signal may comprise recovering corresponding sparse time series (e.g. impulse) signals by finding the peaks in the signal and the corresponding amplitude. In an exemplary embodiment, such amplitudes may correspond to an amount of ingested carbohydrates and/or injected insulin units.

In exemplary embodiments, encoding the sparse time series input signal of the input first data into a dense time series input signal for the first healthcare-related parameter using at least one pharmacokinetic model may comprise encoding sparse time series input signal representing carbohydrate intake and/or bolus insulin into glucose rate of appearance and plasma insulin concentration, respectively, by means of at least one of two compartmental pharmacokinetic models introduced by Hovorka et al. (see Roman Hovorka et al., Nonlinear model predictive control of glucose concentration in subjects with type 1 diabetes, Physiological Measurement, 25:905, 7 2004).

In such embodiments, decoding the dense time series output signal for the first healthcare-relevant parameter into a sparse time series output signal may comprise integrating the area under the curve of the resulting continuous curve. Alternative embodiments may not include a step of decoding the dense time series output signal for the first healthcare-relevant parameter into a sparse time series output signal. For example, use cases such as data augmentation for blood glucose forecasting may not require such signal transformation is (for example in cases when glucose rate of appearance and plasma insulin concentration can be the inputs of the model).

The at least one sparse time series input signal and/or the at least one sparse time series output signal may comprise an impulse signal. Impulse signals in the sense of the present disclosure are non-categorical signals which are zero most of the time, except for isolated samples, for example a few sampling points in a given time period, for example a day, where they take a value corresponding to a healthcare-relevant parameter, for example grams of estimated carbohydrates ingested or injected bolus insulin units.

Alternatively or in addition, the at least one sparse time series input signal and/or the at least one sparse time series output signal may comprise a categorical signal. A categorical signal is a signal that indicates a given value from a limited selection in a given category. For example, a categorical signal may indicate a sex or gender of a patient, a type or sub-type of disease (such as types of heart disease or types of diabetes) or an indicator for a context of a non-categorical measurement such as an activity (for example rest, exercise) during measurement or a device used for performing the measurement.

The input data may further comprise input second data indicative of a second healthcare-relevant parameter, the input second data providing a dense time series input signal for the second healthcare-relevant parameter, wherein the first healthcare-relevant parameter and the second healthcare-relevant parameter are interrelated; and the modified input data may further comprise the input second data. In this case, the method may further comprise determining, in the processing device, output second data by extracting, from the synthetic data, data indicative of the second healthcare-relevant parameter providing a dense time series output signal. With regard to extracting, from the synthetic data, data indicative of a given healthcare-relevant parameter, e.g. the first healthcare-relevant parameter or second healthcare-relevant parameter, data indicative of one given healthcare-relevant parameter may be present in the synthetic data as a closed data set while data indicative of another given healthcare-relevant parameter is present in the synthetic data as another closed data set. In this sense, extraction is achieved by singling out the closed data set indicative of the given healthcare-relevant parameter from the synthetic data.

In the sense of the present disclosure, healthcare-relevant parameters are considered interrelated if they (physiologically) influence one another. Examples include intake events of heart medication and blood pressure, or glucose intake (meal, bolus), insulin bolus and blood glucose.

By generating synthetic data based on input data comprising input first data and input second data, synthetic data referring to heterogenous multivariate time-series data may be generated. Thereby, efficiency may be increased and/or quality of the synthetic data for a given application may be increased, in particular with regard to healthcare applications.

Before the step of determining synthetic data, the method may comprise a pre-processing step comprising at least one of applying linear interpolation to the auxiliary first data and/or the input second data; applying linear extrapolation to the auxiliary first data and/or the input second data; and applying normalization to the input first data, the input second data, and/or the auxiliary first data. Pre-processing may increase quality of determined synthetic data in certain applications. Linear interpolation and/or linear extrapolation may serve to increase quality of data on which synthetic data generation is based by providing additional data points in dense time series data. Exemplary embodiments may comprise determining the synthetic data using machine learning, for example using generative adversarial networks (GANs) such as DoppleGANger, and may comprise a pre-processing step comprising applying normalization to the input first data, the input second data, and/or the auxiliary first data, in particular both to the input second data, and to the input first data or the auxiliary first data.

The input first data may be indicative of at least one of a heart medication event, an exercise event, a meal event and a coronary health event. In this case, the relevant healthcare application may refer to heart disease. Here, the input second data may be indicative of at least one of a heart rate, a blood oxygen saturation, an ejection fraction, a stroke volume, and a cardiac output.

In an alternative embodiment, the input first data is indicative of at least one of an insulin bolus event, a glucose bolus event, a meal event, an amount of meal carbohydrates, an activity event, and insulin pump data. In this case, the relevant healthcare application may refer to diabetes care. Here, the input second data may be indicative of at least one of a blood glucose measurement, a continuous glucose measurement and an insulin pump rate.

In further alternative embodiments, the relevant healthcare application may refer to applications in anesthesia, surgery, or intensive care, for example, and the input first data may be indicative of at least one sparse time series parameter relevant to such application and, if applicable, the input second data may be indicative of at least one dense time series parameter relevant to such application.

The method may further comprise providing, via an output device, output synthetic data comprising the output first data and/or the output second data. Thereby, synthetic data may be provided to be used as input in desired applications. For example, the method may further comprise training a machine learning model using the output synthetic data as training data.

The input data may further comprise input third data indicative of a third healthcare-relevant parameter, the input third data providing a dense time series input signal for the third healthcare-relevant parameter, wherein the first healthcare-relevant parameter, the second healthcare-relevant parameter and the third healthcare-relevant parameter are interrelated. Herein, the modified input data may further comprise the input third data. In some embodiments, the method may comprise determining, in the processing device, output third data by extracting, from the synthetic data, data indicative of the third healthcare-relevant parameter providing a dense time series output signal. The output synthetic data may further comprise the output third data. With regard to the third healthcare-relevant parameter and the corresponding signals and data, the embodiments outlined above with regard to the second healthcare-relevant parameter may apply accordingly.

The input data may further comprise input fourth data indicative of a fourth healthcare-relevant parameter, the input fourth data providing at least one sparse time series input signal for the fourth healthcare-relevant parameter, wherein the first healthcare-relevant parameter, the second healthcare-relevant parameter and the fourth healthcare-relevant parameter (as well as the third healthcare-relevant parameter, if applicable) are interrelated. Herein the method may comprise determining, in the processing device, auxiliary fourth data from the input fourth data, wherein the sparse time series input signal of the input fourth data is encoded into a dense time series input signal for the fourth healthcare-related parameter, the auxiliary fourth data providing the dense time series input signal for the fourth healthcare-related parameter. The modified input data may further comprise the auxiliary fourth data. In some embodiments, the method may comprise determining, in the processing device, output fourth data by extracting, from the synthetic data, data indicative of the fourth healthcare-relevant parameter providing a dense time series output signal for the fourth healthcare-relevant parameter and decoding the dense time series output signal for the fourth healthcare-relevant parameter into at least one sparse time series output signal, the output fourth data providing the sparse time series output signal for the fourth healthcare-relevant parameter. The output synthetic data may further comprise the output fourth data. With regard to the fourth healthcare-relevant parameter and the corresponding signals and data, the embodiments outlined above with regard to the first healthcare-relevant parameter may apply accordingly.

The embodiments outlined above with regard to the first, second, third and/or fourth healthcare-relevant parameters and corresponding signals and data may apply accordingly to any number of further healthcare-relevant parameters, respectively referring to a parameter corresponding to a sparse time series signal or parameter corresponding to a dense time series signal.

According to a further aspect, a synthetic data generation device is provided. The synthetic data generation device comprises an input device, a processing device and an output device. The synthetic data generation device is configured to carry out the method for generating synthetic data for use in healthcare applications. With regard to the synthetic data generation device, the embodiments described above in connection with the method for generating synthetic data for use in healthcare applications may apply accordingly.

According to another aspect, a computer program product is provided, comprising instructions which, when the program is executed by a processing device, cause the processing device to carry out the method for generating synthetic data for use in healthcare applications. The computer program product may be embodied on a computer readable medium, for example a non-transitory computer readable medium. With regard to the computer program product, the embodiments described above in connection with the method for generating synthetic data for use in healthcare applications may apply accordingly.

According to yet another aspect, a method for predicting a healthcare-relevant parameter for a person is provided, the method comprising receiving, in a prediction device, measurement data indicative of a measurement of the healthcare-relevant parameter in a person, predicting at least one future value for the healthcare-relevant parameter of the person using a machine-learning algorithm trained with training data comprising output synthetic data provided by a method for generating synthetic data for use in healthcare applications according to the present disclosure, generating output prediction data indicative of the predicted value for the healthcare-relevant parameter, and outputting the output prediction data via an output device of the prediction device. Machine-learning algorithms suitable for predicting a healthcare-relevant parameter for a person are known as such. Examples include support vector regressor (SVR) (RBF kernel) and LSTM.

The training data may further comprise training measurement data relating to the same healthcare-relevant parameters as the output synthetic data. Thereby, training measurement data may be augmented by synthetic data for training a machine-learning algorithm.

As an alternative or in addition, the measurement data may further be indicative of a further healthcare-relevant parameter or a plurality of healthcare-relevant parameters in the person. Thereby, other parameters may be employed as input in predicting the healthcare-relevant parameter for a person (and, correspondingly in training the machine-learning algorithm). In this case, with regard to each further healthcare-relevant parameter, the training data may comprise output synthetic data, training measurement data or both. Thereby, synthetic data (alone or as part of augmented data) may be used for training a machine-learning algorithm with regard to one or more healthcare-relevant parameters while training with regard to one or more other healthcare-relevant parameters may be performed on the basis of measurement data without (additional) synthetic data. Alternatively, synthetic data (alone or as part of augmented data) may be used for training a machine-learning algorithm with regard to the healthcare-relevant parameter and all of the further healthcare-relevant parameters.

The health care-relevant parameter may be a blood glucose level, the person may be a person with diabetes, and the measurement of the healthcare-relevant parameter may be a continuous glucose measurement. Herein, the measurement data may further be indicative of at least one of the following: an insulin pump rate, an insulin bolus event, a glucose bolus event, a meal event, an amount of meal carbohydrates, an activity event, insulin pump data. Alternatively or in addition, the measurement data may comprise data that is not measured as such but provided, such as one or more of a sex of a subject, an age of a subject, and/or a diabetes type.

In the method the prediction device may be a diabetes management device.

The diabetes management device may a blood glucose monitoring device. The diabetes management device may be a personal mobile device which personal mobile device may be blood glucose monitoring device or not.

In an alternative embodiment, the diabetes management device may be a server in communication with a blood glucose monitoring device. In this case, the diabetes management device may be embodied by software being executed on the server.

Outputting the output prediction data may comprise displaying the output prediction data on a display device. For example, the output prediction data may be displayed on a display of a diabetes management device, such as a blood glucose monitoring device and/or a personal mobile device. As another example, the output prediction data may be displayed on a display of a computing device of a healthcare provider.

Outputting the output prediction data may comprise transmitting the output prediction data to an alarm generation device and, by the alarm generation device, outputting an alarm, if the output prediction data is determined to be indicative of at least one future value for the healthcare-relevant parameter being above a predetermined upper threshold or being below a pre-determined lower threshold. The alarm generation device may be a device of the person for which the health care-relevant parameter is predicted, such as a diabetes management device, blood glucose monitoring device and/or personal mobile device, or may be a device of a healthcare provider.

According to a further aspect, a diabetes management device is provided, the diabetes management device comprising means for carrying out the method for predicting a healthcare-relevant parameter for a person, wherein the healthcare-relevant parameter is a blood glucose level. The diabetes management device may be a single device. For example, the diabetes management device may be a glucose monitor, glucose measurement device (like a BGM or CGM) or personal mobile device of person with diabetes, for example in the form of an application running on a smartphone. Alternatively, the diabetes management device may be provided by a combination of more than one device, for example a glucose monitor, personal mobile device, server device, and/or sensor device functioning together.

Description of further embodiments

Following, embodiments, by way of example, are described with reference to figures. In the figures show:

Fig. 1    a schematic representation of a method for generating synthetic data for use in healthcare applications;
Fig. 2    a schematic representation of a method applying synthetic data;
Fig. 3    a two-dimensional graphical representation of a Gaussian copula for synthetic data generation;
Fig. 4    a graphical representation of a GAN method for synthetic data generation;
Fig. 5    a 24-hour sequence of real data (CGM, meal carbohydrates, insulin) and corresponding encoded impulse signals via Gaussian kernel convolution;
Fig. 6    a synthetically generated 24-hour sequence including CGM and decoded meal carbohydrate and insulin signals;
Fig. 7    a 24-hour sequence of real data (CGM, meal carbohydrates, insulin) and corresponding encoded impulse signals via compartmental pharmacokinetic models; and
Fig. 8    individual RMSE corresponding to different scenarios and glucose predictors.

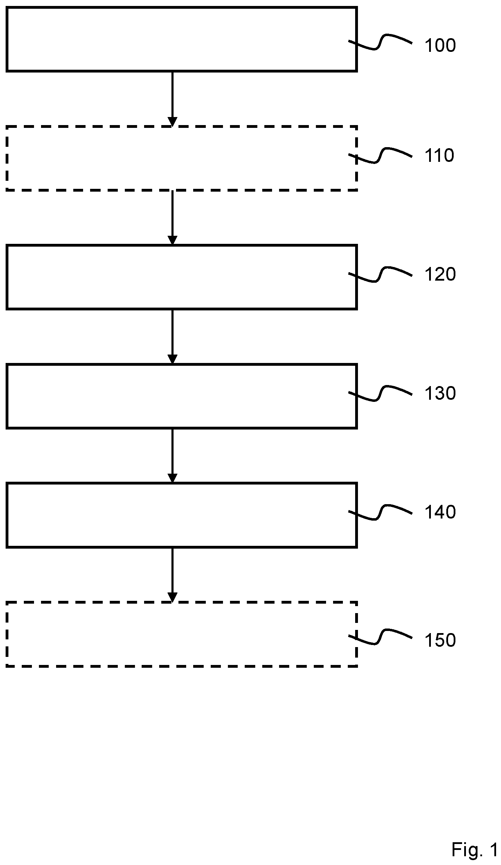

Fig. 1 schematically shows steps of a method for generating synthetic data for use in healthcare applications. In step 100 input data is received via an input device. In the example shown, the input data comprises input first data, input second data and input fourth data.

The input first data is indicative of meal carbohydrates ingested by a person with diabetes. Such data is indicative of a time series signal in which there are short events (carbohydrate intake) with an amplitude, indicating a carbohydrate amount ingested, separated by long periods of a zero amplitude, i.e. no event. Thus, the input first data provides a sparse time series input signal. The input second data is indicative of a continuous glucose monitoring (CGM) signal, i.e. a dense time series input signal, namely a quasi-continuous signal. The input fourth data is indicative of an insulin bolus amount. Thus, such data provides a sparse time series input signal, since data representing an insulin bolus amount, similar to meal carbohydrates, is indicative of a time series signal in which there are short events (insulin bolus application) with an amplitude, indicating a bolus amount, separated by long periods of a zero amplitude, i.e. no event. The first, second and fourth healthcare-relevant parameters are interrelated, i.e., they physiologically influence one another, as for example both carbohydrate intake and insulin bolus directly affect the glucose levels of a person.

In alternative embodiments, there may be further input data indicative of further healthcare-relevant parameters, for example input third data, indicative of a third healthcare-relevant parameter, the input third data providing a dense time series input signal for the third healthcare-relevant parameter, also interrelated with the first, second and fourth healthcare-relevant parameters.

In an embodiment, the method includes a pre-processing step 110 the input data is divided into sequences of fixed length - 24 hours in the exemplary embodiment shown - each providing a number of CGM samples and their corresponding meal and insulin entries. Linear interpolation may be applied to fill CGM gaps. In embodiment directed to glucose prediction (see below) pre-processing may include linear extrapolation applied to avoid data leakage and a sliding window with a length of 24 samples may be used to obtain data batches.

Next, in step 120, auxiliary first data is determined from the input first data and auxiliary fourth data is determined from the input fourth data in a processing device. Herein, the respective time series input signals of the input first data and the input fourth data are encoded into a respective dense time series in-put signal for the first and fourth healthcare-related parameters, the auxiliary first data providing the dense time series input signal for the first healthcare-related parameter (meal carbohydrates) and the auxiliary fourth data providing the dense time series input signal for the fourth healthcare-related parameter (insulin bolus).

In an exemplary embodiment, the encoding is performed using convolution of a normalized gaussian kernel with the sparse time series signals (impulse signals). The normalized gaussian kernel has a standard deviation of 10 and is truncated for values beyond 10 standard deviations. With this kernel, the convolution with the delta signal is a gaussian shifted to the time when the impulse signal occurs (e.g., meal ingestion) and with the amplitude of the impulse signal (e.g., amount of grams).

In an alternative embodiment, the encoding is performed by converting the carbohydrates and bolus insulin into glucose rate of appearance and plasma insulin concentration, respectively, by means of the two compartmental pharmacokinetic models introduced by Hovorka et al. (cf. above).

In step 130, the auxiliary first data, the input second data and the auxiliary fourth data are combined into modified input data.

From the modified input data, synthetic data is determined in step 140 such that the synthetic data closely resembles the modified input data in terms of statistical properties. In an exemplary embodiment, the determining is performed using the Gaussian copula provided by the Synthia library. In an alternative embodiment, the determining is performed using the DoppleGANger GAN architecture. In such alternative embodiment, the pre-processing step 110 may include applying min-max normalization to each feature (i.e., CGM, bolus, and carbohydrate) and scaling them into a range of [0, 1] to facilitate encoding.

The synthetic data may be used as such (summarily representing a multivariate time series) in line with a given application or may be separated into output synthetic comprising output first data, output second data and output fourth data in step 150. Herein, in the processing device, output first data is determined by extracting, from the synthetic data, data indicative of the first healthcare-relevant parameter providing a dense time series output signal for the first healthcare-relevant parameter and decoding the dense time series output signal for the first healthcare-relevant parameter into a sparse time series output signal, the output first data providing the sparse time series output signal for the first healthcare-relevant parameter. Accordingly, the output fourth data is determined by extracting, from the synthetic data, data indicative of the fourth healthcare-relevant parameter providing a dense time series output signal for the fourth healthcare-relevant parameter and decoding the dense time series output signal for the fourth healthcare-relevant parameter into a sparse time series output signal, the output fourth data providing the sparse time series output signal for the fourth healthcare-relevant parameter. The output second data is determined by extracting, from the synthetic data, data indicative of the second healthcare-relevant parameter providing a dense time series output signal for the second healthcare-relevant parameter, the output second data providing the dense time series output signal for the second healthcare-relevant parameter.

In embodiments in which the encoding was performed using kernel convolution, the decoding with regard to the output first data and the output fourth data may comprise recovering the respective sparse time series output signal by finding the peaks in the synthetic data signal and the corresponding amplitude. In the shown embodiment, these amplitudes correspond to the amount of ingested carbohydrates and the injected insulin units. In an exemplary peak detection algorithm, a height of 10 gram and a distance between peaks of two hours may be employed for the carbohydrate signal, and a height of 0.5 U and a two-hour distance for the bolus insulin signal.

In embodiments in which the encoding was performed using pharmacokinetic models, the decoding may comprise integrating the area under the curve of the resulting continuous curve.

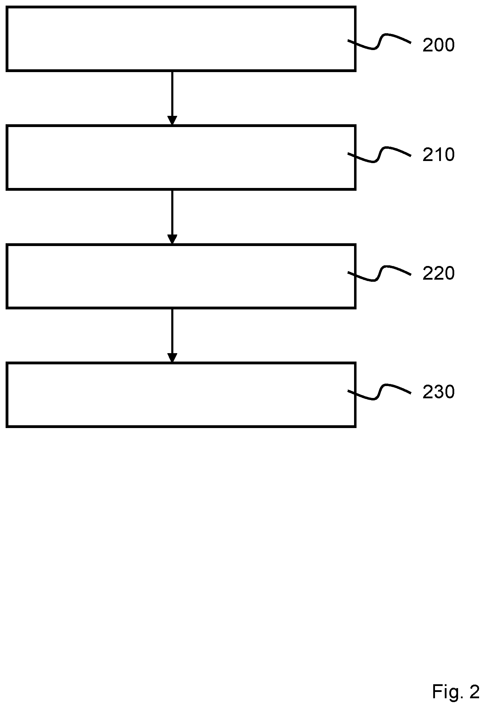

Fig. 2 illustrates steps of a method representing an exemplary use case of the synthetic data generated for example using the method of Fig. 1. In a first step 200, synthetic data obtained on the basis of a real dataset comprising first data indicative of meal carbohydrates ingested by a person with diabetes, second data indicative of a continuous glucose monitoring (CGM) signal, and fourth data indicative of an insulin bolus amount is received.

In step 210, the synthetic data and the real dataset are combined to provide augmented data. In this exemplary embodiment, the real dataset is modified analogous to the encoding step 120 to then be combined with the synthetic data.

Following, the augmented data is used to train an algorithm which forecasts blood glucose levels in the near future (30 minutes ahead) in step 220. In exemplary embodiments, machine learning models support vector regressor (SVR) (RBF kernel) and/or LSTM may be employed herein, for example as laid out below with regard to an evaluation of synthetic data.

In step 230, the trained blood glucose forecasting algorithm is used to predict the blood glucose levels of a person with diabetes from available CGM, carbohydrate and insulin bolus information for that person.

In the following, an exemplary case for evaluating the techniques according to the present disclosure is discussed in detail. It is to be noted that any specific embodiment in such exemplary case with regard to a given step as discussed in the foregoing may accordingly provide an embodiment for a use case of such step in the methods and devices according to the present disclosure.

The exemplary case deals with the problem of generating individualised heterogenous multivariate synthetic time-series data for data augmentation purposes and is not particularly discussed with a view to data privacy, rather focusing on maximizing synthetic data quality.

More specifically, the focus lies on the problem of generating correlated multivariate data including dense/(-quasi-)continuous and sparse/impulse signals, a type of problem commonly found in pharmacokinetic and pharmacodynamic data (e.g., drug boluses vs. drug concentration).

For generating synthetic time-series data, two generative models were chosen, namely Gaussian copula and GANs, being the former a statistics-based method which is computationally very light, and the latter a state-of-the-art machine learning approach which is computationally expensive.

Copulas are probabilistic models based on Sklar's theorem. The copula is a function that combines all marginal distribution into the joint distribution. In the particular case of gaussian copula, the copula is based on the multivariate gaussian distribution (F) and the inverse of the Sklar's theorem. It can be expressed as:

$$C(u_1, \dots, u_d) = F\left(F_1^{-1}(u_1), \dots, F_1^{-1}(u_1)\right),$$

where $F_i$ are the marginal distributions of the different time series. The approach discussed here is based on a particular implementation of Gaussian copula provided by the Synthia library (see above).

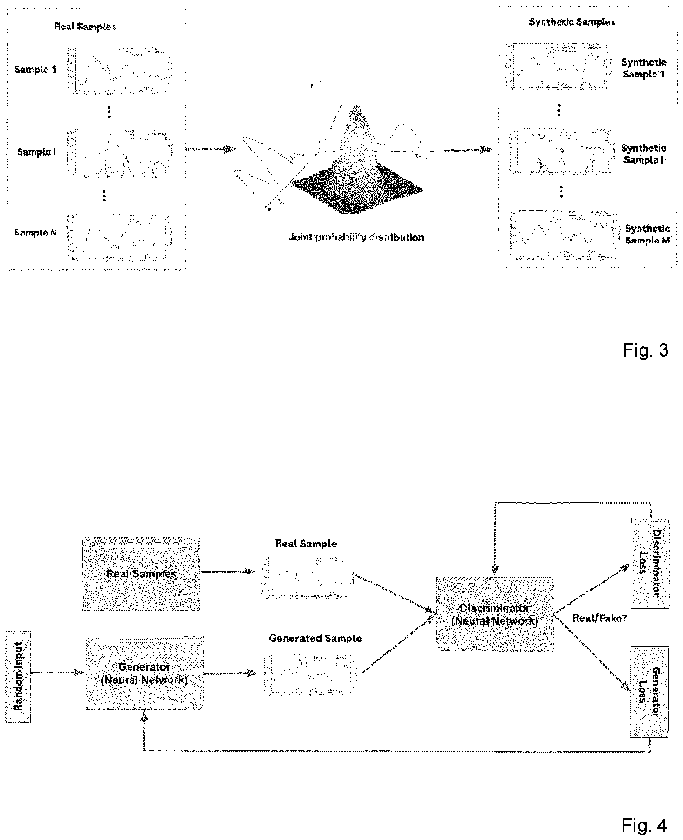

Fig. 3 shows a graphical representation of the Gaussian copula for a two-dimensional example.

Generative adversarial networks (GANs) are a family of methods for generating synthetic data. GANs are composed by two blocks with opposite objectives: the generator and the discriminator. The discriminator is a classifier that distinguishes between real and synthetic data. The role of the generator is to map random noise to valid synthetic data. The discriminator tries to catch the generators synthetic data and the generator tries to create synthetic data in a way that can pass the discriminator, hence they are competing.

Fig. 4 show a generic GAN architecture for synthetic data generation. The GAN architecture of the exemplary case is based on the work proposed by Lin and colleague which is referred to as DoppleGANger (see above). DoppleGANger proposed a framework to generate synthetic time series with matched meta-data. In the present embodiment, only the sub-module of this architecture that generates time series is used. The generator is based on the Long Short-Term Memory (LSTM) cell and uses the Wasserstein loss with gradient penalty to optimize the weights.

The datasets used in the exemplary case are OhioT1DM (Cindy Marling, Razvan Bunescu, *The OhioT1DM dataset for blood glucose level prediction: Update 2020,* Proceedings of the 5th International Workshop on Knowledge Discovery in Healthcare Data co-located with 24th European Conference on Artificial Intelligence, volume 2675, page 71, 2020) and REPLACE-BG (Grazia Aleppo et al., REPLACE-BG: a randomized trial comparing continuous glucose monitoring with and without routine blood glucose monitoring in adults with well-controlled type 1 diabetes, Diabetes Care, 40(4):538-545, 2017). The OhioT1DM dataset was collected in an eight-week clinical trial with 12 adults subject with type 1 diabetes. Participants wore Medtronic Enlite CGM (Medtronic Minimed, Northridge, CA, US) to measure real-time blood glucose levels (every 5 minutes) and recorded daily events, such as meal intake and insulin bolus, using a custom smartphone app. The REPLACE-BG dataset was obtained in a 26-week clinical trial (NCT02258373) with 226 T1D adults on insulin pump therapy. The blood glucose data were measured at a 5-min sampling rate by Dexcom G4 Platinum CGM (Dexcom Inc., San Diego, CA, US) and uploaded to the Tidepool platform (Tidepool, Palo Alto, CA, US), while insulin pump data (including insulin and carbohydrates) were collected by Diasend software (Glooko, Mountain View, CA, US).

There are various errors that can be encountered in clinical T1D datasets. For instance, CGM measurements may contain gaps and discontinuities due to sensor calibration and replacement, signal loss, and errors. Self-reported events may contain manual errors, such as missing entries and misestimation of carbohydrates and insulin boluses. To address such problems, set of pre-processing steps were performed. From the raw data, 24-h sequences of data were generated, corresponding to a repetitive patten. It is noted that other embodiments may use different sequence lengths, for example longer sequences in applications where it is important to capture long-term dependencies in the data. In the exemplary case, the multivariate time series was sliced into daily sequences of 288 CGM samples and their corresponding meal and insulin entries. Then the daily sequences with more than 70% of available CGM measurements and at least one meal event and one insulin bolus event were selected, and linear interpolation to fill the CGM gaps was applied. For the GAN based method (DoppleGANger), min-max normalization to each feature (i.e., CGM, bolus, and carbohydrate) was applied and they were scaled into a range of [0, 1]. Normalization may not be required for the Copula-based method. In glucose prediction, linear extrapolation was performed to avoid data leakage and a sliding window with a length of 24 samples was used to obtain data batches.

The two datasets used in the exemplary case have time-series data of different nature. The CGM is a virtually continuous signal and the carbohydrates and insulin bolus intakes are sparse impulse signals. These two last signals are zero most of the time, except for few sampling points a day where they take a value corresponding to the grams of estimated carbohydrates or the injected bolus insulin units. Such kind of sparse impulse signals, as it is the case for categorical variables, is problematic with the known synthetic data generation methods. In the exemplary case the impulse signals were convert into a continuous signals. Also recovering the impulse signals from the generated synthetic data was explored.

The first encoding method of the exemplary case is a convolution of a given kernel with the impulse signals (e.g., carbohydrates and bolus signals). In particular, the kernel used is a normalized gaussian kernel with a standard deviation of 10 and truncated for values beyond 10 standard deviations. With this kernel, the convolution with the delta signal is a gaussian shifted to the time when the impulse signal occurs (e.g., meal ingestion) and with the amplitude of the impulse signal (e.g., amount of grams). Other kernels were also tested (e.g., Butterworth, staircase). Once the synthetic data has been generated, the corresponding impulse signals may be recovered by finding the peaks in the signal and the corresponding amplitude. In the exemplary case, these amplitudes correspond to the amount of ingested carbohydrates and the injected insulin units. For the peak detection algorithm, a height of 10 gram and a distance between peaks of two hours was employed for the carbohydrate signal, and a height of 0.5 U and a two-hour distance for the bolus insulin signal.

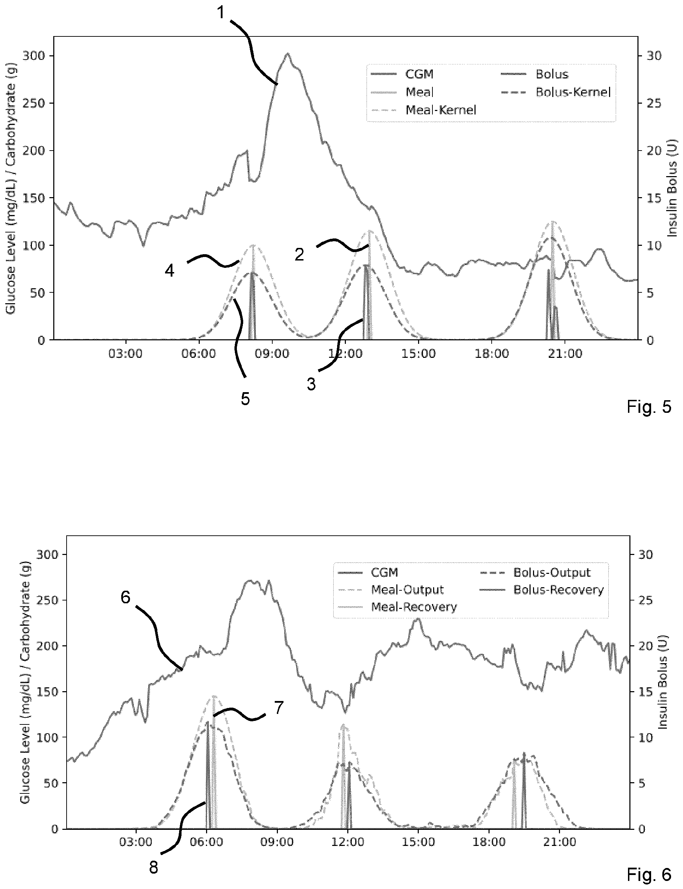

Fig. 5 shows an example of a 24-hour sequence of real data (CGM 1, meal carbohydrates 2, insulin 3) with the corresponding encoded impulse signals for meal carbohydrates 4 and insulin 5 via Gaussian kernel convolution. Fig. 6 shows an example of a synthetically generated 24-hour sequence including the CGM 6 and the decoded meal carbohydrate 7 and insulin 8 signals.

The second encoding method of the exemplary case consists in converting the carbohydrates and bolus insulin into glucose rate of appearance and plasma insulin concentration respectively by means of two compartmental pharmacokinetic models introduced by Hovorka et al. (see above). In this case, the decoding step is more complex, requiring integrating the area under the curve of the resulting continuous curve. Since, for the blood glucose forecasting data augmentation problem discussed below, the decoding phase for this signal transformation is not required (i.e., glucose rate of appearance and plasma insulin concentration can be the inputs of the model), this was not implemented in the exemplary case.

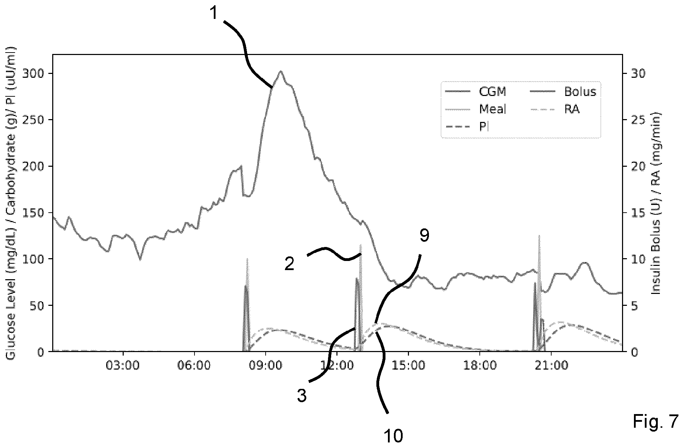

Fig. 7 shows an example of a 24-hour sequence of real data (CGM 1, meal carbohydrates 2, insulin 3) with the corresponding encoded impulse signals for meal carbohydrates 9 and insulin 10 via the compartmental pharmacokinetic models.

To evaluate the quality of the generated synthetic data, a total of 30 glycemic metrics, which are known as such in the art, were used, which includes: mean, standard deviation, median, minimum, maximum, coefficient of variation, and the first and third quartiles of glucose levels; time in range (TIR), TIR (70-140 mg/dL), time below range (TBR), and time above range (TAR), TAR (> 250 mg/dL); risk index, low blood glucose index, and high blood glucose index, average daily risk range (ADRR), and glycemic risk index (GRI); mean absolute glucose change (MAG) and mean amplitude of glucose excursions (MAGE); continuous overall net glycemic action over 24 hours (CONGA24); mean of daily differences (MODD); American Diabetes Association estimated HbA1c (eA1c); glucose management index (GMI); J-index of glucose, and five zones of control-variability grid analysis (CVGA). In addition, seven metrics were employed to evaluate the fidelity and similarity of daily scenarios, including, percentage of total daily insulin (TDI), total daily carbohydrates (TDC), average meals per day (AMD), total daily carbohydrates, postprandial glucose peak time, postprandial glucose peak level, and mean carbohydrates amount for breakfast, lunch, and dinner. Other five metrics including cross-correlation between CGM traces, Jensen-Shannon distance, principal component analysis (PCA) and t-distributed stochastic neighbor embedding (t-SNE) for visual evaluation purposes, and post-hoc recurrent neural networks (RNN) predictive and classification scores. In the selected glucose forecasting use case, the root mean square error (RMSE) was used to evaluate the performance of machine learning predictors on the following scenarios, train on real, test on real (TRTR); train on synthetic, test on real TSTR; and train on augmented, test on real (TATR).

After recovering the meal and insulin signals from the generated synthetic data, as described above, the 42 diabetes care metrics presented above were applied to the real and synthetic data. To compare the distributions of the real and synthetic data, D'Agostino's K2 tests were first used to confirm normality and F -tests to test the equality of variances. Then, independent t-tests were applied to compute p values corresponding to a null hypothesis that the means of the two groups are equal. For the sake of space, a subset of the 42 metrics is discussed here. The selected subset, which are common metrics for evaluating glycemic control, include: mean and standard deviation of CGM, total daily insulin (TDI), total daily carbohydrates (TDC), average meals per day (AMD), time in range (TIR), time below range (TBR), and time above range (TBR). Table 1 and 2 present the results corresponding to OhioT1DM dataset and REPLACE-BG dataset, respectively, for the Gaussian copula method. Herein, Table 1 shows population-level metrics (mean±SD) for OhioT1DM dataset (n=12) corresponding to real data and synthetic data generated with Gaussian copula, and Table 2 shows population-level metrics (mean±SD) for REPLACE-BG dataset (n=226) corresponding to real data and synthetic data generated with Gaussian copula. It is worth noting that the generated synthetic datasets with Gaussian copula are statistically equivalent to their real counterparts (p » 0.05). However, this is not the case for the GAN-based method. To demonstrate data generalization (i.e., synthetic days are not copies of real days), the mean cross-correlation between real CGM data and synthetic CGM data was calculated. The relatively small cross-correlation (less than 0.5 for both datasets) and visual inspection indicates a good degree of generalization.

**Table 1:**

| Metrics | Mean CGM (mg/dL) | SD CGM (mg/dL) | TDI (U) | TDC (g) | AMD | %TIR | %TBR | %TAR |
|---|---|---|---|---|---|---|---|---|
| **Mean±SD (Real)** | 158.6 ±16.2 | 57.4 ±5.8 | 33.0 ±18.4 | 169.8 ±67.6 | 3.62 ±1.15 | 64.0 ±9.72 | 3.47 ±2.53 | 32.4 ±10.8 |
| **Mean±SD (Synthetic)** | 157.4 ±17.4 | 54.3 ±6.0 | 31.2 ±17.2 | 167.6 ±70.9 | 3.32 ±0.65 | 64.9 ±10.0 | 2.90 ±2.73 | 32.1 ±11.4 |
| **p value** | 0.86 | 0.18 | 0.81 | 0.94 | 0.46 | 0.84 | 0.61 | 0.95 |

**Table 2:**

| Metrics | Mean CGM (mg/dL) | SD CGM (mg/dL) | TDI (U) | TDC (g) | AMD | %TIR | %TBR | %TAR |
|---|---|---|---|---|---|---|---|---|
| **Mean±SD (Real)** | 158.2 ±19.6 | 58.7 ±10.6 | 21.7 ±10.4 | 156.9 ±57.4 | 4.22 ±1.15 | 64.3 ±11.0 | 3.84 ±3.01 | 31.8 ±12.0 |
| **Mean±SD (Synthetic)** | 158.0 ±19. | 7 57.4 ±10.5 | 20.1 ±10.3 | 151.2 ±57.5 | 4.06 ±0.75 | 64.5 ±11.1 | 3.52 ±3.16 | 31.9 ±12.3 |
| **p value** | 0.94 | 0.29 | 0.13 | 0.41 | 0.34 | 0.89 | 0.49 | 0.96 |

To test whether the synthetic data maintains the multivariate dependencies of the real data, the cross-correlation between the time series was calculated after applying the encoding described above. Table 3 presents the results of such correlations for Gaussian copula. Table 3 shows correlation (mean±SD) between real and synthetic multivariate time series generated with Gaussian copula (Synthia). For each combination of the variables, it is observed that the cross-correlation of synthetic data is close to that of real data for both the OhioT1 DM dataset and the REPLACE-BG dataset.

**Table 3:**

| Variables | Glucose vs Bolus | Glucose vs Meal | Bolus vs Meal |
|---|---|---|---|
| **OhioT1DM** | | | |
| **Mean±SD (Real)** | 0.49 ±0.0 | 0.49 ±0.0 | 0.63 ±0.05 |
| **Mean±SD (Synthetic)** | 0.48 ±0.03 | 0.48 ±0.03 | 0.60 ±0.04 |
| **REPLACE-BG** | | | |
| **Mean±SD (Real)** | 0.48 ±0.02 | 0.49 ±0.02 | 0.62 ±0.05 |
| **Mean±SD (Synthetic)** | 0.48 ±0.02 | 0.49 ±0.02 | 0.62 ±0.0 |

To evaluate the utility of the generated synthetic data, forecast of blood glucose levels in the near future (30 minutes ahead) was included in the exemplary case. Evaluation was with a view to the performance of blood glucose forecasting algorithm trained with synthetic data (i.e., TSTR) instead of real data (i.e., TRTR) and a view to increasing, by augmenting a real dataset with synthetic data, the performance of the blood glucose forecasting algorithm (i.e., TATR). The OhioT1DM dataset was used. Two machine learning models, support vector regressor (SVR) (RBF kernel) and LSTM, known as such, were employed. The OhioT1DM dataset comes with a training (6 weeks) and testing set (2 weeks). The last 20% of training data are used as validation sets, and hyperparameter tuning using grid search was performed. For LSTM, early stopping with a patience of 20 was applied to model training with a total of 500 epochs, aiming to avoid overfitting. An Adam optimizer with a learning rate of 0.001 and a batch size of 128 was used to perform gradient descent and update model weights. For comparing TRTR versus TSTR, for each subject in the dataset the same number of synthetic days as real days in the training set was generated. To evaluate the potential benefit of synthetic data augmentation on blood glucose forecasting (i.e., TRTR vs. TATR), a reduced data scenario was assumed where only seven-day of training data per subject are available. The duration of most commercial CGM devices is 7-10 days, on which window length selection was based. From the 7 days of real data, personalized synthetic datasets of lengths 7, 14, 21, and 28 days were generated. By adding these synthetic data into the reduced training datasets (i.e., 7 days), the performance of blood glucose prediction (evaluated as RMSE) when trained on augmented data was compared versus training on real data with the same length of the augmented datasets (i.e., 7, 14, 21, and 28 days).

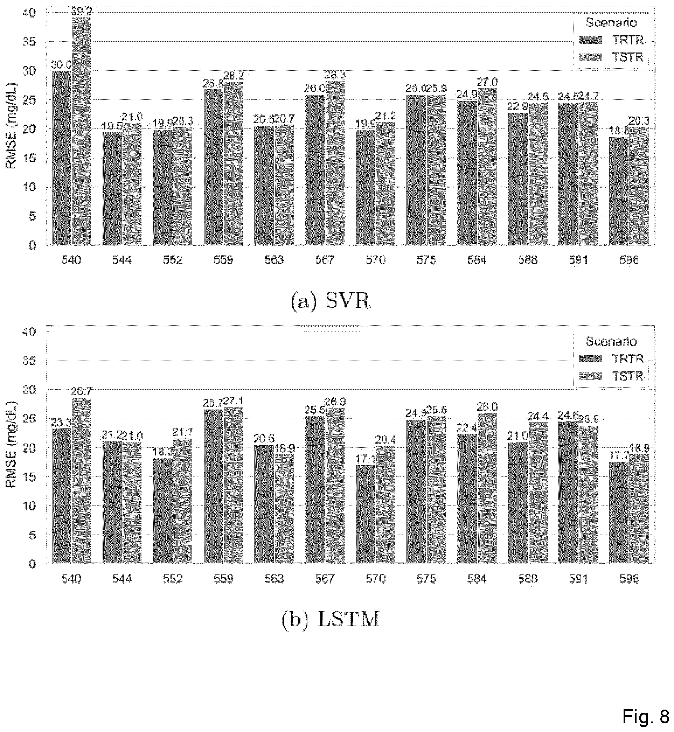

With regard to evaluating the utility of the generated synthetic data, the OhioT1DM dataset was employed. Fig. 8 shows the RMSE plots for the two evaluated machine learning glucose forecasting models, SVR (RBF kernel) and LSTM, under two scenarios, TRTR (left column for each individual) and TSTR (right column for each individual). First, for each individual, the same amount of synthetic data (i.e., number of days) was generated as that of real data. Note that this result corresponds to using the Gaussian kernel encoding/decoding method for generating the synthetic data. For TRTR, the personalized models was trained using all the real training data and tested on the corresponding testing sets. For TSTR, the synthetic data were used in model training and the models were tested on the same testing sets used for TRTR. It can be observed that the TSTR performance (SVR: RMSE = 25.1 mg/dL; LSTM: RMSE = 23.6 mg/dL) is statistically non-inferior to that of TRTR (RMSE = 23.3 mg/dL, p = 0.34; LSTM: RMSE = 21.9 mg/dL, p = 0.22). It is worth noting that, in the LSTM case, the use of synthetic training data reduced RMSE for subjects 544, 563, and 591, when compared with the TRTR scenario.

Table 4 shows the results corresponding to the data argumentation experiments using the Gaussian kernel encoding/decoding method as Population RMSE (mean $\pm$ SD) corresponding to SVR and LSTM algorithms when evaluated on the OhioT1DM dataset (n=12) with Gaussian kernel encoding and decoding (TRTR vs TATR). More specifically, the following is reported according to the notation DX, where D is the number of days and X is R for real and S for synthetic: 7R; 7R+7S; 14R; 7R+14S; 21R; 7R+21S; 28R; 7R+28S. It is worth noting that, for both prediction algorithms, there is a trend towards improvement when the amount of synthetic data increases. Similarly, Table 5 shows the results corresponding to using the metabolic model-based encoding method as Population RMSE$\pm$SD corresponding to SVR and LSTM algorithms when evaluated on the OhioT1DM dataset (n=12) with metabolic model encoding (TRTR vs TATR). Note that, in terms of improvements, this last results are consistent with results from Table 4. However, the performance for the SVR is significantly worse.

**Table 4:**

| Data Partitioning | 7R | 7R+7S | 14R | 7R+14S | 21R | 7R+21S | 28R | 7R+28S |
|---|---|---|---|---|---|---|---|---|
| SVR | 28.8$\pm$6.0 | 28.1$\pm$3.2 | 24.8$\pm$3.5 | 27.3$\pm$4.1 | 24.2$\pm$3.3 | 27.9$\pm$3.8 | 24.$\pm$3.1 | 27.6$\pm$4.2 |
| LSTM | 24.8$\pm$3.5 | 24.2$\pm$2.6 | 22.9$\pm$2.8 | 24.2$\pm$3.2 | 21.9$\pm$2.7 | 24.2$\pm$3.1 | 21.5$\pm$3.1 | 24.4$\pm$3.1 |

**Table 5:**

| Data Partitioning | 7R | 7R+7S | 14R | 7R+14S | 21R | 7R+21S | 28R | 7R+28S |
|---|---|---|---|---|---|---|---|---|
| SVR | 37.8$\pm$7.2 | 36.9$\pm$6.6 | 30.8$\pm$4.9 | 35.6$\pm$6.6 | 29.2$\pm$3.9 | 35.7$\pm$5.8 | 28.1$\pm$4.0 | 35.4$\pm$6.6 |
| LSTM | 24.4$\pm$4.3 | 25.1$\pm$3.8 | 22.7$\pm$3.0 | 25.0$\pm$3.5 | 22.1$\pm$2.8 | 25.9$\pm$3.8 | 21.9$\pm$3.0 | 25.8$\pm$3.8 |

The features disclosed in this specification, the figures and / or the claims may be material for the realization of various embodiments, taken in isolation or in various combinations thereof.

**Claims**

1. A method for generating synthetic data for use in healthcare applications, the method comprising

- receiving, via an input device, input data comprising

- input first data indicative of a first healthcare-relevant parameter, the input first data providing at least one sparse time series input signal for the first healthcare-relevant parameter,

- determining, in a processing device, auxiliary first data from the input first data, wherein the sparse time series input signal of the input first data is encoded into a dense time series input signal for the first healthcare-related parameter, the auxiliary first data providing the dense time series input signal for the first healthcare-related parameter; and

- determining, in the processing device, synthetic data, from modified input data comprising the auxiliary first data, such that the synthetic data closely resembles the modified input data in terms of statistical properties.

2.  The method according to claim 1, further comprising:

    - determining, in the processing device, output first data by extracting, from the synthetic data, data indicative of the first healthcare-relevant parameter providing a dense time series output signal for the first healthcare-relevant parameter and decoding the dense time series output signal for the first healthcare-relevant parameter into a sparse time series output signal, the output first data providing the sparse time series output signal for the first healthcare-relevant parameter.

3.  The method according to claim 1 or 2, wherein the synthetic data is determined using a statistics-based method.

4.  The method according to claim 1 or 2, wherein the synthetic data is determined using machine learning.

5.  The method according to any one of the preceding claims, wherein determining the auxiliary first data comprises encoding the sparse time series input signal of the input first data into a dense time series input signal for the first healthcare-related parameter using Kernel Convolution and/or at least one pharmacokinetic model.

6.  The method according to any one of the preceding claims, wherein

    - the input data further comprises input second data indicative of a second healthcare-relevant parameter, the input second data providing a dense time series input signal for the second healthcare-relevant parameter, wherein the first healthcare-relevant parameter and the second healthcare-relevant parameter are interrelated; and
    - the modified input data further comprises the input second data

7.  The method according to any one of the preceding claims, comprising, before the step of determining synthetic data, a pre-processing step comprising at least one of the following:

    - applying linear interpolation to the auxiliary first data and/or the input second data;
    - applying linear extrapolation to the auxiliary first data and/or the input second data; and
    - applying normalization to the input first data, the input second data, and/or the auxiliary first data.

8.  The method according to any one of the preceding claims, further comprising providing, via an output device, output synthetic data comprising the output first data and/or the output second data.

9.  The method according to claim 8, further comprising training a machine learning model using the output synthetic data as training data.

10. A synthetic data generation device, comprising an input device, a processing device and an output device, wherein the synthetic data generation device is configured to carry out the method according to any one of the claims 1 to 9.

11. A computer program product, comprising instructions which, when the program is executed by a processing device, cause the processing device to carry out the method according to any one of the claims 1 to 9.

12. A method for predicting a healthcare-relevant parameter for a person, the method comprising:

    - receiving, in a prediction device, measurement data indicative of a measurement of the healthcare-relevant parameter in a person;
    - predicting at least one future value for the healthcare-relevant parameter of the person using a machine-learning algorithm trained with training data comprising output synthetic data provided by the method according to any one of the claims 1 to 16;
    - generating output prediction data indicative of the predicted value for the healthcare-relevant parameter; and
    - outputting the output prediction data via an output device of the prediction device.

13. The method according to claim 12, wherein

    - the healthcare-relevant parameter is a blood glucose level;
    - the person is a person with diabetes; and
    - the measurement of the healthcare-relevant parameter is a continuous glucose measurement.

14. The method according to claim 13, wherein the prediction device is a diabetes management device.

15. A diabetes management device, comprising means for carrying out the method according to any one of claims 12 to 14.

Fig. 1

Fig. 2

Fig. 3

Fig. 4

Fig. 5

Fig. 6

Fig. 7

(a) SVR

(b) LSTM

Fig. 8

# EUROPEAN SEARCH REPORT

**Application Number**

EP 24 38 2580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHU TAIYU ET AL: "GluGAN: Generating Personalized Glucose Time Series Using Generative Adversarial Networks", IEEE JOURNAL OF BIOMEDICAL AND HEALTH INFORMATICS, IEEE, PISCATAWAY, NJ, USA, vol. 27, no. 10, 2 May 2023 (2023-05-02), pages 5122-5133, XP011951085, ISSN: 2168-2194, DOI: 10.1109/JBHI.2023.3271615 [retrieved on 2023-05-03] * The whole document, in particular: Abstract; Pages 5124, 5125, 5129 - 5131; Figures, 1, 6, 7. * ----- | 1-15 | INV. G16H10/60 G16H50/50 G16H50/70 |

**TECHNICAL FIELDS SEARCHED (IPC)**

G16H
G06N

The present search report has been drawn up for all claims

| Place of search | Date of completion of the search | Examiner |
|---|---|---|
| Munich | 12 November 2024 | Nagele, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022235618 A1


**Non-patent literature cited in the description**

- **RICHARD J CHEN et al.** Synthetic data in machine learning for medicine and Healthcare. *Nature Biomedical Engineering*, 2021, vol. 5 (6), 493-497
- **SIDDHARTH BISWAL**. Generating longitudinal electronic health records using conditional variational autoencoders. *Machine Learning for Healthcare Conference*, 2021, 260-282
- **DAVID MEYER et al.** Copula-based synthetic data augmentation for machine-learning emulators. *Geoscientific Model Development*, 2021, vol. 14 (8), 5205-5215
- **FABI PREZJA et al.** Deepfake knee osteoarthritis x-rays from generative adversarial neural networks deceive medical experts and offer augmentation potential to automatic classification. *Scientific Reports*, 2022, vol. 12 (1), 1-16
- **FODIL BENALI et al.** Synthetic complex data generation using copula. *International Workshop on Design, Optimization, Languages and Analytical Processing of Big Data (DOLAP)*, 2021, 51-60

- **PHILIPP WENDLAND et al.** Generation of realistic synthetic data using multimodal neural ordinary differential equations. *NPJ digital medicine*, 2022, vol. 5 (1), 1-10
- **DAVID MEYER ; THOMAS NAGLER**. Synthia: multidimensional synthetic data generation in python. *Journal of Open Source Software*, 2021, vol. 6 (9), 2863
- **ZINAN LIN et al.** Using GANs for sharing networked time series data: Challenges, initial promise, and open questions. *Proceedings of the ACM Internet Measurement Conference*, 2020, 464-483
- **MARTIN ARJOVSKY et al.** *Wasserstein GAN*, 06 December 2017
- **ROMAN HOVORKA et al.** Nonlinear model predictive control of glucose concentration in subjects with type 1 diabetes. *Physiological Measurement*, 2004, vol. 25 (905), 7
- **GRAZIA ALEPPO et al.** REPLACE-BG: a randomized trial comparing continuous glucose monitoring with and without routine blood glucose monitoring in adults with well-controlled type 1 diabetes. *Diabetes Care*, 2017, vol. 40 (4), 538-545